# EUROPÄISCHE PATENTSCHRIFT

(11) **EP 0 861 058 B1**
(45) Veröffentlichungstag und Bekanntmachung des Hinweises auf die Patenterteilung: **04.09.2002**
(21) Anmeldenummer: 97923048.9
(22) Anmeldetag: 09.05.1997
(51) Int. Cl.: A61F 2/46

(54) **HÜFTPFANNE UND CHIRURGISCHES INSTRUMENT ZUM EINSETZEN DERSELBEN**
ACETABULAR PROSTHESIS AND SURGICAL INSTRUMENT FOR INSERTION THEREOF
PROTHESE COTYLOIDIENNE ET INSTRUMENT CHIRURGICAL POUR SON IMPLANTATION

(30) Priorität: 09.05.1996 DE 29608453 U
(43) Veröffentlichungstag der Anmeldung: 02.09.1998
(73) Patentinhaber: Waldemar Link GmbH & Co., 22339 Hamburg (DE)
(72) Erfinder: KELLER, Arnold, D-23863 Kayhude (DE)
(74) Vertreter: Glawe, Delfs, Moll & Partner
(86) Internationale Anmeldenummer: EP9702399
(87) Internationale Veröffentlichungsnummer: WO97042915

(56) Entgegenhaltungen:
- EP-A- 0 303 810
- EP-A- 0 453 694
- WO-A-94/21199
- WO-A-95/11641
- FR-A- 2 281 095
- FR-A- 2 663 840
- FR-A- 2 676 172
- FR-A- 2 721 502
- US-A- 5 112 338
- US-A- 5 169 399
- US-A- 5 171 313

## Beschreibung

Zum Einsetzen von Hüftpfannen-Endoprothesen in das ggf. mit einem Zementbett versehene Acetabulum sind Instrumente gebräuchlich, die am Ende eines Griffstabs einen Haltekopf tragen, der zum Halten der Pfanne in einer vorbestimmten Relativposition zum Instrument einen in die Höhlung der Pfanne passend eingreifenden Ansatz und einen sich auf den Rand der Pfanne auflegenden Kragen aufweist. Die zum Eindrücken der Pfanne in das Acetabulum erforderliche Kraft läßt sich damit leicht auf die Pfanne übertragen. Wünschenswert ist aber, daß die Pfanne auch in der entgegengesetzten Kraftrichtung und in Drehrichtung fest an dem Instrument gehalten ist und sie sich ohne Übertragung wesentlicher Kräfte davon wieder lösen läßt.

Zu diesem Zweck weist ein bekanntes Einsetzinstrument (FR-A-2721502), das der Bildung des Oberbegriffs des Anspruchs 1 zugrundeliegt, einen in die Hüftpfanne greifenden Ansatz, der genau an die Form der die Höhlung bildenden Öffnung angepaßt ist, und achsparallel am Kragen angeordnete Haltestifte auf, die in als achsparallele Bohrungen ausgeführte Halteöffnungen an der Stirnseite der Hüftpfanne fassen. Diese wird durch den Ansatz und die Haltestifte mittels Reibungskräften an dem Instrument gehalten. Zum Abnehmen drückt ein gegen Federkraft mittels eines Betätigungsorgans axial verschieblicher Kolben das Instrument von der Hüftpfanne ab. Nachteilig ist, daß die Hüftpfanne nur über den unsicheren Reibeingriff am Instrument gehalten ist, der sehr stark bemessen werden muß, damit auch im Falle des Zusammenkommens ungünstigster Toleranzabweichungen die angestrebte Haltekraft erreicht wird. Zum Lösen muß eine die Haltekraft überwindende, verhältnismäßig große Kraft aufgebracht werden, die in der Regel größer ist als die angestrebte Haltekraft. Je größer die Kraft ist, die zum Lösen aufgebracht werden muß, um so schwerer ist es, das Instrument ruhig zu halten.

Ein gleichfalls durch Reibkraft die Prothese haltendes Einsetzinstrument ist aus US-5169399 bekannt. Es umfaßt einen an die Form der Höhlung der Hüftpfanne angepaßten Kopf, dessen Hälften durch Federkraft auseinander und gegen die Innenwand der Hüftpfanne gepreßt werden. Zum Lösen des Instruments von der Hüftpfanne werden die Kopfhälften mit einem Betätigungshebel aufeinander zu bewegt, so daß der Kontakt mit der Hüftpfanne aufgehoben wird. In Anbetracht des geringen Reibungskoeffezienten der Innenfläche einer Hüftpfanne muß bei diesem Einsetzinstrument die Kraft, mit der die Kopfhälften gegen die Innenfläche der Hüftpfanne gepreßt werden und die als Lösekraft beim Abnehmen des Instruments aufgebracht werden muß, größer als die angestrebte Haltekraft sein. Die Nachteile dieser Vorrichtung entsprechenden daher den oben genannten. Dazu kommt der nur unbefriedigende Schutz gegen ungewolltes Verdrehen der Hüftpfanne auf dem Instrument.

Bei einem anderen bekannten Einsetzinstrument (WO94/21199) greifen radial angeordnete Haltevorsprünge in Halteöffnungen an der Innenseite der Hüftpfanne ein. Auf das Instrument, das einen zentrierend in eine Bodenöffnung der Prothese passenden Ansatz aufweist, ist ein Zwischenstück gesteckt, an dem quer zur Öffnungsrichtung bewegliche unter Federkraft in die Halteöffnungen eingreifende und durch ein Betätigungsorgan daraus zurückziehbare Haltevorsprünge angeordnet sind. Diese Anordnung ist nachteilig, weil die Öffnungen in der Innenfläche der Prothese im allgemeinen unerwünscht sind und eine größere Wandstärke der Prothese an anatomisch ungünstiger Stelle erfordern.

Zum Halten von Hüftpfannenfräsern sind Instrumente bekannt (US-5171313, FR-2281095), die mit radial angeordneten Haltestiften in Öffnungen an der Innenseite eines Fräskopfes fassen, die mittels eines Betätigungsorgans gegen Federkraft zurückziehbar sind. Die Verwendung solcher Instrumente zum Halten von Implantaten wäre aus dem im vorstehenden Absatz genannten Grund nachteilig.

Bei einem Instrument zum Lösen implantierter Hüftpfannenprothesen (US-5112338) greifen radial angeordnete Haltevorsprünge in Schlitze an der Stirnseite der Hüftpfanne. Da die Schlitze nach vorne offen sind, ist es nicht möglich, die Hüftpfanne an dem Instrument zu halten.

Zur Implantation einer Halterungsschale für eine Gelenkpfanne, die im Acetabulum zementlos mit Hilfe von Dornen verankert wird, die auf elastischen Zungen angeordnet sind, ist ein Einsetzinstrument bekannt (EP-A-453694), das speziell in solcher Weise ausgebildet ist, daß die Zungen während des Einsetzens von dem Instrument nach innen gezogen werden, damit die Dornen die Einsetzbewegung der Halterungsschale an die gewünschte Position nicht stören. Wenn diese erreicht ist, löst man das Werkzeug, damit die Zungen wieder die äußere Lage einnehmen und die Dornen zur Fixierung der Halterungsschale in das umgebende Gewebe dringen. Da die Zungen und die mit dem Instrument zusammenwirkenden Öffnungen in der Tiefe der Halterungsschale angeordnet sind, gibt die bekannte Anordnung keinen Hinweis, wie man den Eingriff des Instruments in die Höhlung der Pfanne vermeiden kann.

Ausgehend von dem aus FR-A-2721502 bekannten Instrument liegt der Erfindung die Aufgabe zugrunde, eine aus Einsetzinstrument und Pfanne bestehende Anordnung zu schaffen, bei der das Instrument stirnseitig an der Pfanne angreift und trotzdem sicher reproduzierbare Haltekräfte ausgeübt werden können und bei der das Instrument sich leicht von der Pfanne lösen läßt. Die erfindungsgemäße Lösung besteht in den Merkmalen des Anspruchs 1.

Die Hüftpfanne weist in ihrer Randstirnfläche wenigstens zwei Haltevertiefungen auf, die in bezug auf die Öffnungsrichtung hinterschnitten sind. Die Öffnungsrichtung ist diejenige Richtung, in der sich der Hüftpfanne öffnet. Es ist auch die Richtung, in der aufgrund der Richtung der zusammenwirkenden Oberflächenteile von Instrument und Hüftpfanne das Instrument von der Hüftpfanne zu entfernen ist. Das ist im allgemeinen die Richtung normal zu der Ebene, in welcher die Randstirnfläche liegt.

Das Instrument weist einen in die Öffnung oder an den Öffnungsrand der Hüftpfanne zentrierend passenden Ansatz auf. Außerdem hat es mindestens zwei Haltevorsprünge, die den Haltevertiefungen der Hüftpfanne örtlich zugeordnet sind und demgemäß in die Haltevertiefung eingefügt werden können. Sie weisen einen Haken auf, der in den Hinterschnitt der Haltevertiefung eingreifen kann. In dieser Stellung sind sie arretierbar. Mittels eines besonderen Betätigungsorgans sind sie aus der Arretierungsstellung zurückziehbar. In der Regel sind die zwei oder mehr Haltevorsprünge und -vertiefungen regelmäßig über den Umfang verteilt, damit deren Arretierungskräfte sich gegenseitig ausgleichen. Der Vorteil dieser Anordnung besteht darin, daß die Hüftpfanne sicher auch gegen Ausübung beträchtlicher Kräfte am Instrument gehalten ist, solange die Arretierung der Haltevorsprünge in den Haltevertiefungen nicht gelöst ist, daß aber nach Lösung der Arretierung keinerlei Zugkräfte mehr beim Abnehmen des Instruments von der Hüftpfanne auf die letztere ausgeübt werden. Die Arretierung braucht nicht in der Art einer festen Verriegelung ausgeführt zu sein, obwohl dies möglich ist; im Regelfall genügt es, wenn die Haltevorsprünge durch Federkraft in der Arretierungsstellung gehalten werden, wobei die Federkraft so hoch gewählt wird, daß sie zum Festhalten der Hüftpfanne am Instrument unter allen praktisch vorkommenden Bedingungen ausreicht.

Das Betätigungsorgan, mit dem die Arretierung gelöst wird, ist zweckmäßigerweise bei einem Handgriff des Instruments an einer in normaler Greifstellung zugänglichen Stelle angeordnet. Dadurch soll es dem Arzt gestattet werden, das Instrument zu lösen, ohne seine Handstellung zu verändern. Dadurch wird vermieden, daß unerwünschte Relativbewegungen auf die Hüftpfanne übertragen werden und deren Position versehentlich geändert wird.

Zweckmäßigerweise verlaufen die Haltevorsprünge des Instruments etwa in Öffnungsrichtung und die Haken sind (im Verhältnis zu ihrer Hauptrichtung) seitlich gerichtet. Diese Ausführungsform ist dann besonders einfach, wenn die Haltevertiefungen als winklig zur Öffnungsrichtung verlaufende Bohrungen ausgebildet sind, wobei die winklige Schrägung vorzugsweise in Radialrichtung nach außen oder innen verläuft. Entsprechend weisen auch die Haken der Haltevorsprünge und die Richtung der Arretierungsbewegung der Haltevorsprünge nach außen oder innen.

Die Eingriffsicherheit kann ferner dadurch vergrößert werden, daß die Bohrungen mit einer Umfangsriefelung, beispielsweise in der Form eines Gewindes, versehen sind. Dies gilt insbesondere dann, wenn der seitliche Ansatz des Haltevorsprungs eine Kante oder mehrere Kanten aufweist, die mit der Riefelung zusammenwirken.

Damit die Haltevorsprünge des Instruments quer zur Öffnungsrichtung der Hüftpfanne bewegt werden können, sind sie zweckmäßigerweise an Gleitstücken angeordnet, die in im wesentlichen radialen Gleitführungen des Werkzeugkopfs geführt sind. Für ihre Bewegung kann eine Getriebeanordnung vorgesehen sein, die die Gleitstücke mit einer Betätigungswelle verbindet, die in einer zentrisch an dem Kopf angeordneten, diesen mit dem Handgriff verbindenden Stange drehbar gelagert ist und die das Betätigungsorgan trägt, das seitlich aus der Verbindungsstange herausragt. Die Arretierungsfeder kann auf die Gleitstücke wirken oder an der Betätigungswelle angreifen.

Die Erfindung wird im folgenden näher unter Bezugnahme auf die Zeichnung erläutert, die ein vorteilhaftes Ausführungsbeispiel veranschaulicht. Darin zeigen:
- Fig. 1: den Kopf des Instruments und eine Hüftpfanne im Längsschnitt gemäß Linie I-I der Fig. 2,
- Fig. 2: einen Querschnitt durch den Kopf des Instruments gemäß Linie II-II der Fig. 1,
- Fig. 3: eine Seitenansicht des Instrumentenkopfs in Richtung III der Fig. 2 und
- Fig. 4: eine Gesamtansicht des Instruments.

Das Instrument besteht aus dem Handgriff 1, dem Instrumentenkopf 2 und einer diese beiden Teile verbindenden Stange 3, an der das Betätigungsorgan 4 so angeordnet ist, daß es bei normaler Handstellung leicht in Pfeilrichtung mittels eines Fingers gegen Federkraft betätigt werden kann. Durch diese Betätigung kann die Verbindung zu dem zuvor von dem Instrument gehaltenen Hüftpfanne gelöst werden.

Der Instrumentenkopf weist einen zylindrischen Ansatz 5 auf, dessen Durchmesser mit einem gewissen Spiel passend zur Öffnungsgröße und -form der Pfanne 6 bemessen ist und in die Öffnung eingesetzt wird. Er weist ferner einen Kragen 8 auf, der dazu bestimmt ist, auf die Stirnfläche 9 der Pfanne 6 aufgesetzt zu werden. Dadurch wird das Instrument auf die Öffnungsrichtung 10 der Pfanne ausgerichtet, die in den dargestellten Beispielen mit der Mittellinie der Pfanne und des Instruments übereinstimmt.

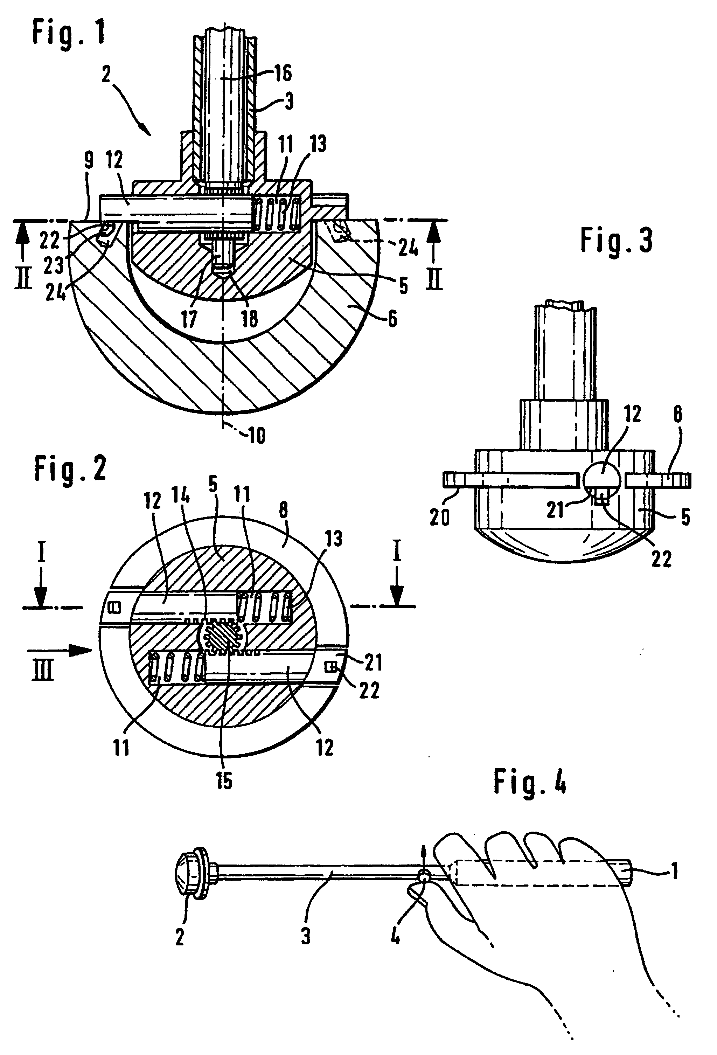

Im Instrumentenkopf sind zwei Bohrungen 11 enthalten, die mittelpunktsymmetrisch zueinander parallel in einer lotrecht zur Mittellinie 10 verlaufenden Ebene liegen und je ein Gleitstück 12 enthalten, das durch eine Feder 13 nach außen gedrängt ist. Auf ihrer der Mittellinie zugewandten Seite sind die Gleitstücke 12 als Zahnstangen 14 ausgebildet, die mit einem zentral angeordneten Ritzel 15 zusammenwirken. Das Ritzel 15 ist am Ende einer Welle 16 angeordnet, die in der hohl ausgebildeten Stange 3 verläuft und mit dem Betätigungsorgan 4 verbunden ist. Am äußersten Ende ist sie mit einem Zapfen 17 versehen, der in einer Bohrung 18 des Instrumentenkopfs gelagert ist. In ihrem übrigen Verlauf wird die Welle von der Innenfläche der Stange 3 gelagert. Durch Betätigung des Betätigungsorgans 4 in Richtung des in Fig.4 gezeigten Pfeils wird das Ritzel so gedreht, daß die Gleitstücke 12 gegen die Wirkung der Federn 13 in den Instrumentenkopf zurückgezogen werden. Läßt man das Betätigungsorgan 4 los, so gelangen diese Teile in die in Fig. 2 dargestellte Endstellung.

In der dargestellten Ausführungsform tragen die Gleitstücke in einer mit der Unterfläche 20 des Kragens 8 fluchtenden Ebene 21 je einen Haltevorsprung 22, dessen Hauptrichtung etwa parallel zur Mittelachse 10 verläuft und der am Ende einen Haken 23 trägt, der etwa radial nach außen weist. In der Stirnfläche 9 der zugehörigen Pfanne 6 sind an entsprechender Umfangsstelle Bohrungen 24 vorgesehen, die ein wenig schräg radial nach außen verlaufen und dadurch, wie in Fig. 1 dargestellt, einen Hinterschnitt (in bezug auf die Achse 10) bilden, in den der Haken 23 eingreifen kann. Zum Verbinden der beiden Teile werden durch Betätigung des Betätigungsorgans 4 die Gleitstücke 12 zurückgezogen. In ihrer inneren Endstellung befinden sich die Haltevorsprünge 22 bei etwa derselben Radialposition wie die Öffnungen der Bohrungen 24. Es läßt sich daher leicht die Stellung finden, in der das Instrument und die Pfanne so zusammenpassen, daß die Haltevorsprünge 22 in die Bohrungen 24 eingreifen können. Danach läßt man das Betätigungsorgan los; die Gleitstücke 12 gleiten etwa radial nach außen, wodurch die Haken 23 in Eingriff mit dem Hinterschnitt der Bohrungen 24 gelangen. Die Pfanne sitzt dann am Instrument fest, weil die Haken 23 in der Haltestellung durch die Federn 13 arretiert sind. Die Neigung der Bohrungen 24 und die Kraft der Federn 13 lassen sich leicht so bemessen, daß die Haltekraft, mit der die Haken 23 in der Haltestellung arretiert sind, für jeden vernünftigerweise vorkommenden Anwendungsfall hinreicht. Die Haltekraft der Haken 23 in den schrägen Bohrungen 24 kann durch eine Riefelung (beispielsweise Gewindeschnitt) der Bohrungen noch verbessert werden. Es versteht sich, daß die Entfernung der Bohrungen 24 voneinander nicht größer ist als der Maximalabstand der Haken 23 voneinander. Sobald die Pfanne eingesetzt ist oder aus anderen Gründen vom Instrument gelöst werden soll, wird wieder das Betätigungsorgan 4 betätigt; die Haltevorsprünge 22 ziehen sich dabei radial zurück und das Instrument kann von der Pfanne entfernt werden, ohne daß irgendwelche störenden Kräfte auf diese ausgeübt werden.

Das Ausführungsbeispiel zeigt, daß die Bewegungsrichtung der Haltevorsprünge 22 nicht genau radial sein muß, sondern lediglich eine hinreichende Radialbewegungskomponente aufzuweisen braucht. Die Bewegung braucht auch nicht geradlinig geführt zu sein; es könnte sich vielmehr auch um eine irgendwie geartete Schwenkbewegung handeln.

## Patentansprüche

1. Anordnung bestehend aus einer Hüftpfannenprothese (6) und einem Einsetzinstrument (1 bis 4), das einen in die Öffnung der Hüftpfannenprothese (6) zentrierend eingreifenden Ansatz (5), wenigstens zwei in Haltevertiefungen (24) in der Randstirnfläche (9) der Hüftpfannenprothese (6) eingreifenden Haltevorsprünge (22) und ein auf die Halteverbindung zwischen dem Einsetzinstrument (1 bis 4) und der Hüftpfannenprothese (6) einwirkendes Betätigungsorgan (4) aufweist, **dadurch gekennzeichnet, daß** die Haltevertiefungen (24) hinterschnitten sind und die Haltevorsprünge (22) einen in den Hinterschnitt fassenden Haken (23) aufweisen, wobei die Haltevorsprünge (22) mittels des Betätigungsorgans (4) quer zur Öffnungsrichtung zwischen einer Haltestellung, in der sie mit dem Hinterschnitt haltend zusammenwirkend arretierbar sind, und einer den Hinterschnitt freigebenden Stellung verschiebbar sind.

2. Anordnung nach Anspruch 1, **dadurch gekennzeichnet, daß** zur Arretierung der Haltevorsprünge (22) im Hinterschnitt eine Feder (13) vorgesehen ist.

3. Anordnung nach Anspruch 1 oder 2, **dadurch gekennzeichnet, daß** das Betätigungsorgan (4) bei einem Handgriff (1) des Instruments an einer in normaler Greifstellung zugänglichen Stelle angeordnet ist.

4. Anordnung nach einem der Ansprüche 1 bis 3, **dadurch gekennzeichnet, daß** das Instrument einen sich auf die Randstirnfläche (9) der Hüftpfanne (6) aufsetzenden Kragen (8) aufweist.

5. Anordnung nach einem der Ansprüche 1 bis 4, **dadurch gekennzeichnet, daß** die Haltevertiefungen (24) als winklig zur Öffnungsrichtung (10) verlaufende Bohrungen ausgebildet sind.

6. Anordnung nach Anspruch 5, **dadurch gekennzeichnet, daß** die Bohrungen (24) eine Umfangsriefelung, beispielsweise in der Form eines Gewindes, aufweisen.

7. Anordnung nach einem der Ansprüche 1 bis 6, **dadurch gekennzeichnet, daß** der den Ansatz (5) und ggf. den Kragen (8) bildende Kopf (2) des Instruments im wesentlichen radiale Gleitführungen (11) mit darin geführten Gleitstükken (12) enthält, an denen die Haltevorsprünge (22) angeordnet sind.

8. Anordnung nach Anspruch 7, **dadurch gekennzeichnet, daß** eine Getriebeanordnung (14, 15) zwischen den Gleitstücken (12) und einer Betätigungswelle (16) vorgesehen ist, die in einer zentrisch an dem Kopf (2) angeordneten, diesen mit dem Handgriff (1) verbindenden Stange (3) drehbar gelagert ist und das seitlich aus der Verbindungsstange (3) herausragende Betätigungsorgan (4) trägt.

## Claims

1. An assembly comprising an acetabular prosthesis (6) and an installation instrument (1 to 4) which has an extension (5) engaging in a centering manner in the opening of the acetabular prosthesis (6), at least two retaining projections (22) engaging in retaining recesses (24) in the marginal end face (9) of the acetabular prosthesis (6) and an actuating member (4) acting on the retaining connection between the installation instrument (1 to 4) and the acetabular prosthesis (6), **characterised in that** the retaining recesses (24) are undercut and the retaining projections (22) have a hook (23) engaging in the undercut, wherein the retaining projections (22) can be displaced by means of the actuating member (4) transversely to the opening direction between a retaining position, in which they can be located to co-operate retentively with the undercut, and a position releasing the undercut.

2. An assembly according to Claim 1, **characterised in that** a spring (13) is provided to locate the retaining projections (22) in the undercut.

3. An assembly according to Claim 1 or 2, **characterised in that** the actuating member (4) is arranged on a handle (1) of the instrument at a position which is accessible in a normal gripping position.

4. An assembly according to any one of Claims 1 to 3, **characterised in that** the instrument has a collar (8) fitted on to the marginal end face (9) of the acetabulum (6).

5. An assembly according to any one of Claims 1 to 4, **characterised in that** the retaining recesses (24) are in the form of bores extending angularly to the opening direction (10).

6. An assembly according to Claim 5, **characterised in that** the bores (24) have peripheral grooving, for example in the form of a screw-thread.

7. An assembly according to any one of Claims 1 to 6, **characterised in that** the head (2) of the instrument forming the extension (5) and optionally the collar (8) contains substantially radial sliding guides (11) with slide members (12) guided therein, on which the retaining projections (22) are disposed.

8. An assembly according to Claim 7, **characterised in that** a gear arrangement (14,15) is provided between the slide members (12) and an actuating shaft (16) which is rotatably mounted in a rod (3) arranged centrally on the head (2) and connecting the latter to the handle (1), and which carries the actuating member (4) projecting laterally from the connecting rod (3).

## Revendications

1. Dispositif constitué d'une prothèse cotyloïdienne (6) et d'un instrument d'insertion (1 à 4), qui comporte un appendice (5) s'engageant, de manière centrée, dans l'ouverture de la prothèse cotyloïdienne (6), au moins deux saillies de maintien (22) s'engageant dans des renfoncements de maintien (24) de la surface frontale de bordure (9) de la prothèse cotyloïdienne (6), et un organe d'actionnement (4) agissant sur la liaison de maintien entre l'instrument d'insertion (1 à 4) et la prothèse cotyloïdienne, **caractérisé en ce que** les renfoncements de maintien (24) sont détalonnés et les saillies de maintien (22) comportent un crochet (23) s'engageant dans le détalonnage, les saillies de maintien (22) pouvant coulisser, au moyen de l'organe d'actionnement (4), transversalement à la direction d'ouverture, entre une position de maintien dans laquelle elles peuvent être bloquées en coopération de maintien avec le détalonnage, et une position qui dégage le détalonnage.

2. Dispositif selon la revendication 1, **caractérisé en ce que** pour bloquer les saillies de maintien (22), il est prévu un ressort (13) dans le détalonnage.

3. Dispositif selon la revendication 1 ou 2, **caractérisé en ce que** l'organe d'actionnement (4) est disposé dans une poignée (1) de l'instrument, en un emplacement accessible en position normale de prise.

4. Dispositif selon l'une des revendications 1 à 3, **caractérisé en ce que** l'instrument comporte une collerette (8) placée sur la surface frontale de bordure (9) de la prothèse cotyloïdienne (6).

5. Dispositif selon l'une des revendications 1 à 4, **caractérisé en ce que** les renfoncements de maintien (24) sont réalisés sous la forme de perçages qui s'étendent en angle par rapport à la direction d'ouverture (10).

6. Dispositif selon la revendication 5, **caractérisé en ce que** les perçages (24) présentent un rainurage périphérique, par exemple sous la forme d'un filetage.

7. Dispositif selon l'une des revendications 1 à 6, **caractérisé en ce que** la tête (2) de l'instrument, qui forme l'appendice (5) et éventuellement la collerette (8), contient des glissières (11) sensiblement radiales avec des coulisseaux (12) guidés à l'intérieur, sur lesquels sont disposées les saillies de maintien (22).

8. Dispositif selon la revendication 7, **caractérisé en ce qu'**il est prévu, entre les coulisseaux (12) et un arbre d'actionnement (16), une transmission (14, 15) qui est montée tournante dans une tige (3) disposée au centre sur la tête (2) et reliant celle-ci à la poignée (1), et qui porte l'organe d'actionnement (4) ressortant sur le côté de la tige de liaison (3).
